# EUROPEAN PATENT SPECIFICATION

(11) **EP 2 964 069 B1**
(45) Date of publication and mention of the grant of the patent: **27.05.2020**
(21) Application number: 14720719.5
(22) Date of filing: 26.02.2014
(51) Int. Cl.: A61B 34/30, A61B 18/22, A61B 34/00, A61B 18/20

(54) **A ROBOTIC MANIPULATOR SYSTEM**
ROBOTERMANIPULATORSYSTEM
SYSTÈME DE ROBOT MANIPULATEUR

(30) Priority: 26.02.2013 US 201361769453 P
(43) Date of publication of application: 13.01.2016
(73) Proprietor: Kabakci, Ahmet Sinan, 06370 Ankara (TR); Saglam, Remzi, Ankara (TR); Koruk, Erhan, Ankara (TR)
(72) Inventor: Kabakci, Ahmet Sinan, 06370 Ankara (TR); Saglam, Remzi, Ankara (TR); Koruk, Erhan, Ankara (TR)
(74) Representative: Grünecker Patent- und Rechtsanwälte PartG mbB
(86) International application number: PCT/TR2014/000052
(87) International publication number: WO 2014/133476

(56) References cited:
- EP-A1- 1 986 563
- WO-A2-2011/008922
- US-A- 6 120 433
- US-A- 6 120 433
- US-A1- 2007 083 098
- US-A1- 2007 083 098
- US-A1- 2007 142 970
- US-A1- 2007 142 970
- US-A1- 2011 022 229
- US-A1- 2011 022 229
- US-A1- 2012 065 470
- US-A1- 2012 065 470

## Description

### Technical Field

The present invention is related to a robotic manipulator system, which is used for manipulating commercially available flexible endoscopes.

### Prior Art

Generally, there have been attempts to perform a minimally invasive surgical (MIS) procedure. Such MIS techniques are aimed at reducing the amount of extraneous tissue that is damaged during diagnostic or surgical procedures, thereby reducing patient recovery time, discomfort, and deleterious side effects. A common form of such procedures is endoscopy, for example, which is used for minimally invasive inspection and surgery inside the patient's body. To perform such MIS procedures, a surgeon needs a special medical instrument (i.e., endoscope). The surgeon passes these instruments through a small incision of a body wall to a surgical site and manipulates the medical instrument from outside the body wall by sliding the medical instrument in and out through the body wall, rotating and pivoting the medical instrument against the body wall.

However, it has been found that a high level of dexterity is required to accurately control such a medical instrument. And, the surgeon has no flexibility of tool replacement. Additionally, he or she experiences difficulty in approaching the surgical site through the incision. The length and construction of differing medical instruments reduces the surgeon's ability to feel forces exerted by the surgical site on the medical instruments. Further, human hands typically have at least a minimal amount of tremor. The tremor further increases the difficulty of performing minimally invasive surgical procedures. So, only a relatively small number of surgeries have been performed due to limitations in medical instruments, techniques and the surgical training.

Patent application no US2012004668A1 discloses a robotic catheter system including a controller with a master input device. An instrument driver is in communication with the controller and has a guide instrument interface including a plurality of guide instrument drive elements responsive to control signals generated, at least in part, by the master input device.

An elongate guide instrument has a base, distal end, and a working lumen, wherein the guide instrument base is operatively coupled to the guide instrument interface. The guide instrument includes a plurality of guide instrument control elements operatively coupled to respective guide drive elements and secured to the distal end of the guide instrument. The guide instrument control elements are axially moveable relative to the guide instrument such that movement of the guide instrument distal end may be controlled by the master input device.

US 2012/0065470 A1 discloses a robotic system for steerable tip endoscopes including a support arm, an endoscope gripping assembly rotatably connected to the support arm and a translation assembly operatively connected to the support arm.

WO 2011/008922 A2 discloses a robotic catheter system with an instrument driver in communication with a controller and having an elongate instrument interface including a plurality of instrument drive elements responsive to control signals generated by using input through a master input device operatively coupled to the controller and an elongate instrument having a guide instrument base operatively coupled to the guide instrument interface.

EP 1 986 563 A1 discloses an apparatus for positioning a distal end of a working instrument at a body site such as a lumen includes a guide instrument and a working instrument disposed with a lumen of the guide instrument.

### Brief Description of the Invention

The object of the invention is to provide a robotic manipulator system for manipulating flexible endoscopes. This object is achieved by the robotic manipulator system for maneuvering an insertion tube with the features of claim 1. Dependent claims disclose further preferred embodiments.

In the present disclosure, a robotic manipulator system for maneuvering an existing medical instrument to a desired location within a target zone during a medical procedure is provided. Said robotic manipulator system comprises, at least one control unit capable of receiving an operator input; at least one robotic manipulator, in communication with said control unit and responsive to said operator input, said robotic manipulator including, at least one controller, a rotation mechanism in communication with said at least one controller and being rotated about a first axis, a horizontal movement unit in communication with said at least one controller and being displaced along a first path, and a deflection actuator capable of receiving a portion of the existing instrument, said deflection actuator being in communication with said at least one controller and being displaced along a second path; wherein displacement of said deflection mechanism along said second path causes deflection of a distal end of the existing medical instrument, wherein said control unit comprises at least one control console and said control console comprises at least one display device for displaying system parameters, wherein the robotic manipulator system comprises at least one laser fiber which is inserted through a working channel of the flexible uretero-renoscope, wherein a robotic arm of the robotic manipulator further comprises at least one laser fiber actuator for actuating said laser fiber, and in that said laser fiber actuator comprises at least one laser fiber holder for holding a laser fiber connection adapter and at least one movement unit for moving said laser fiber holder with respect to the flexible uretero-renoscope , and wherein an advancement distance of the laser fiber is indicated on the display device as the distance from a distal end of the flexible uretero-renoscope to the tip of the laser fiber during movement of the laser fiber actuator, and wherein a foot pedal is provided to control emission of laser radiation, and software of the robotic manipulator controls the emission of the laser by controlling the foot pedal such that, when the surgeon presses the foot pedal for laser shooting, the robot does not enable the laser if the tip of the laser fiber is close to the distal end of the flexible uretero-renoscope to avoid any damage of the uretero-renoscope.

Another object of the present disclosure is to provide a robotic manipulator system which eliminates the requirement of surgeon holds the manipulator during the operation.

Another object of the present disclosure is to provide a robotic manipulator system which keeps the surgeon away from X-ray radiation area.

Another object of the present disclosure is to provide a robotic manipulator system which reduces the time of operation.

Another object of the present disclosure is to provide a robotic manipulator system which operates automatically and ensures precision and sensibility.

### Description of the Drawings

Figure 1 shows an exemplary embodiment of the control unit.
Figure 2 shows an exemplary embodiment of the robotic manipulator.
Figure 3 shows exemplary embodiment of the control unit at another view.
Figure 4 shows exemplary embodiment of the robotic manipulator at another view.
Figure 5 shows endoscope connection of the robotic manipulator.
Figure 6 shows robotic arm of the robotic manipulator.
Figure 7 shows the monitor and controller of the control unit.
Figures 8 and 9 show the connection detail of endoscope connection and robotic arm of the robotic manipulator.

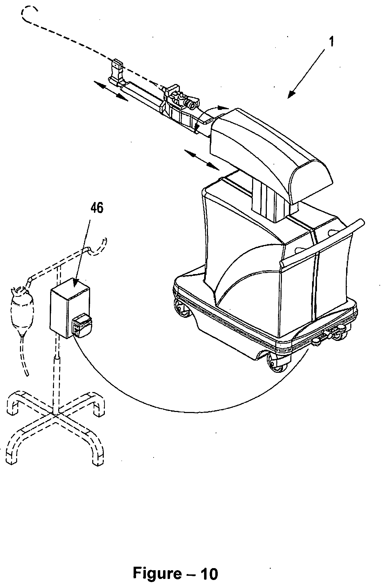

Figure 10 shows another exemplary embodiment of the robotic manipulator.

The reference numbers as used in figures may possess the following meanings.

| | |
|---|---|
| Robotic manipulator | (1) |
| Control Unit | (2) |
| Flexible endoscope | (3) |
| Connection unit | (4) |
| Robotic arm | (5) |
| Deflection actuator | (6) |
| Laser fiber actuator | (7) |
| Horizontal movement unit | (8) |
| Vertical movement unit | (9) |
| Control console | (10) |
| Seat | (11) |
| Monitor | (12) |
| Pedal | (13) |
| Control means | (14) |
| Deflection handle | (14a) |
| Rotation and insertion handle | (14b) |
| Handle lever | (14c) |
| Deflection lever coupler | (15) |
| Laser fiber holder | (16) |
| Laser fiber connection adapter | (17) |
| Deflection lever | (18) |
| Connection rod | (19) |
| Display device | (20) |
| Upper plate | (21) |
| Carrier plate | (22) |
| Linear bearing | (23) |
| Endoscope holder | (24) |
| Arm flange | (25) |
| Bearing system | (26) |
| Speed reducer | (27) |
| Motor flange | (28) |
| Rotational motor | (29) |
| Bearing assembly | (30) |
| Ball screw nut flange | (31) |
| Rotation feedback sensor | (32) |
| Ball screw nut | (33) |
| Belt and pulley | (34) |
| Horizontal movement motor | (35) |
| Lover plate | (36) |
| Cover | (37) |
| Slide linear bearing | (38) |
| Linear slide | (39) |
| Signal connector | (40) |
| Precision wheel | (41) |
| Deflection control sensor | (42) |
| Electromagnetic break | (43) |
| Timing belt | (44) |
| Arm rest | (45) |
| Irrigation pump | (46) |

### Detailed Description of the Invention

In the treatment of the kidney stones, several different methods are used. In one of said methods, flexible endoscopes are used for the treatment. Flexible endoscopes (flexible uretero-renoscope or fURS) comprises a long and relatively thin insertion tube, which is inserted to patient's body through the ureter, and at least one means for controlling the flexible endoscope. Said tube comprises a flexible portion at its tip, wherein said flexible portion is able to deflect. Deflection of the flexible tip is controlled by a deflection lever placed on the handle. By moving said deflection lever to up or down, flexible tip of the endoscope is deflected to one direction or opposite.

In the surgical operations for treating kidney stones, said insertion tube is inserted to the patient's body through the ureter until the flexible tip extends to the kidney. When the tip is entered to the kidney, flexible tip reaches to the kidney stones and said kidney stone is fragmented or dusted (e.g. by using a laser). In order to reach the kidney stones, many maneuvers are applied to the flexible endoscope, such as rotating the flexible endoscope, inserting the insertion tube into the patient and deflecting the tip. Since said surgical operation requires 45-60 minutes, said manoeuvres, as well as the requirement of staying standing is troublesome for the operator. Therefore, according to the present disclosure, a robotic manipulator system for manipulating flexible endoscopes is provided.

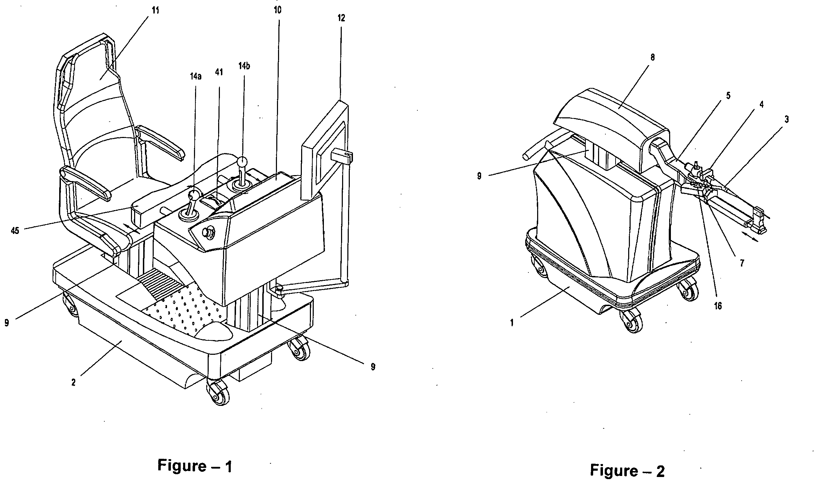

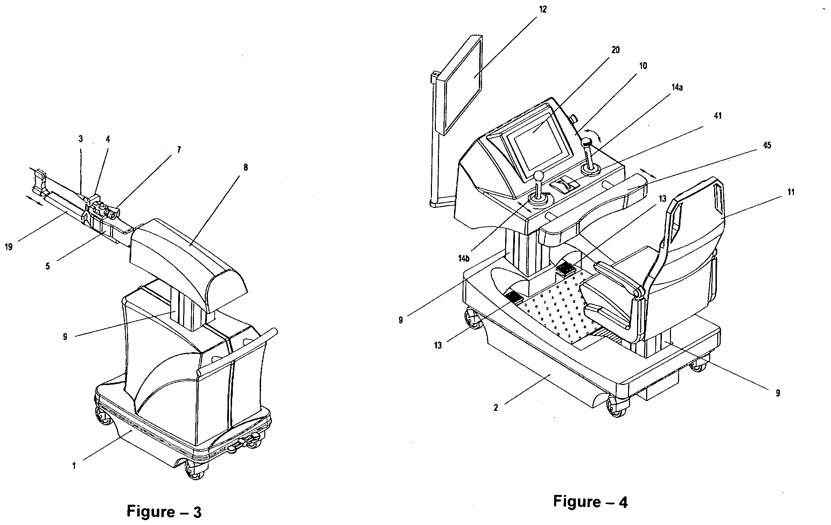

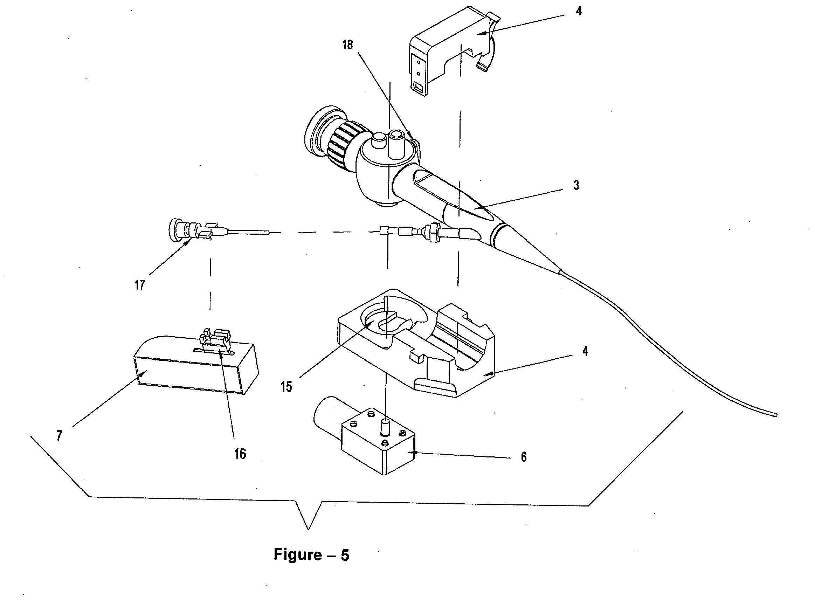

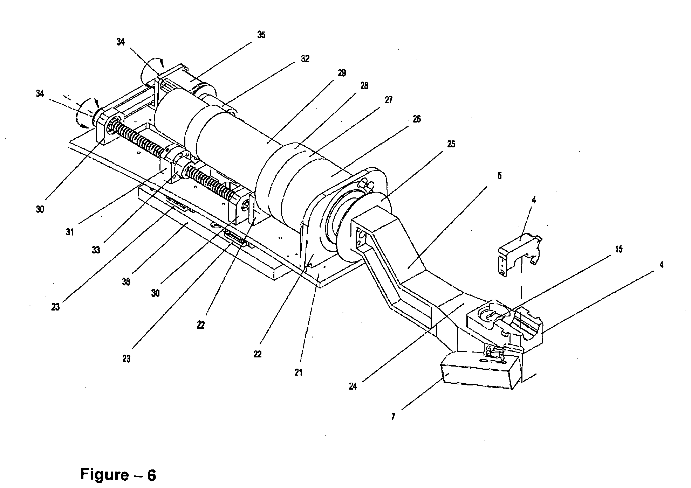

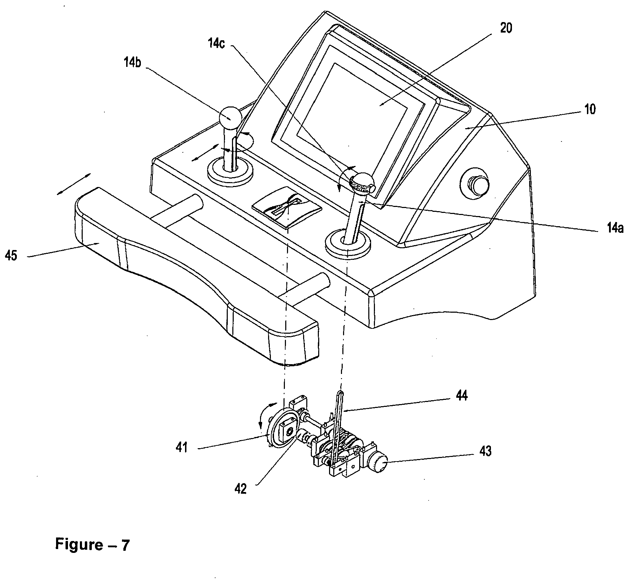

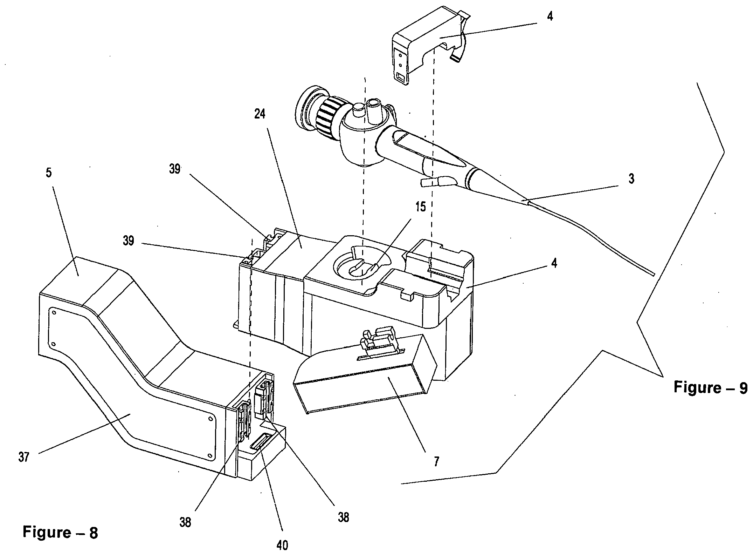

Exemplary embodiments of the robotic manipulator system of the present disclosure are shown in figures 1-9. Said robotic manipulator system comprises, and at least one control unit (2) capable of receiving an operator input and at least one robotic manipulator (1), in communication with said control unit (2) and responsive to said operator input. Said robotic manipulator (1) comprises, at least one controller; a rotation mechanism in communication with said at least one controller by said input and being rotated about a longitudinal axis of the insertion tube; a horizontal movement unit (8) in communication with said at least one controller by said input and being displaced along a first path which is parallel or coincides with the longitudinal axis of the tube, and a deflection actuator (6) capable of receiving a portion of the existing instrument, said deflection actuator (6) being in communication with said at least one controller by said input and being displaced along a second path. Displacement of said deflection mechanism along said second path causes deflection of a distal end of the existing medical instrument. Said medical instrument may be a flexible endoscope (3) or an uretero-renoscope. Said robotic manipulator (1) and control unit (2) may be connected to each other by a cable (such as Ethernet cable, USB cable) or a wireless connection.

Preferably, said robotic manipulator comprises at least one robotic arm (5), which makes the rotation movement and which is in connection with said medical instrument. Said robotic arm (5) comprises at least one connection unit (4), which fixes said medical instrument to said robotic arm (5).

In an exemplary embodiment, horizontal movement unit (8) may be a unit placed on robotic manipulator (1) and moves the robotic arm (5) (as shown in figures). In another exemplary embodiment, said horizontal movement unit (8) is placed on robotic manipulator (1) and moves the robotic manipulator (1) with respect to the patient. In another exemplary embodiment, horizontal movement unit (8) is placed on patient's side (for example at operation table) and moves patient with respect to the robotic manipulator (1).

In a preferred embodiment of the present disclosure, said control unit (2) comprises at least one control means (14) for controlling the movement of the robotic manipulator (1). Said control means (14) comprises at least one deflection handle (14a) and at least one rotation and insertion handle (14b). Said deflection handle (14a) comprises at least one handle lever (14c), for performing the deflection movement. Said handle lever (14c) is similar to the deflection lever (18) of the flexible endoscope (3). Therefore, the surgeons, who are accustomed to flexible endoscope (3), are easily able to be use the deflection handle (14a). Said rotation and insertion handle (14b) is preferably in the form of a stick that both rotates and moves forward/backward. By rotating the rotation and insertion handle (14b), said robotic arm (5) rotates. By moving the rotation and insertion handle forward/backward, said horizontal movement unit (8) moves flexible endoscope (3) relative to the patients.

In another preferred embodiment of the present disclosure, said connection unit (4) comprises at least one deflection lever coupler (15) for gripping the deflection lever (18). Therefore, precise movement of the deflection lever (18) is ensured.

During the kidney stone treatment by laser, at least one laser fiber is inserted through insertion tube of the flexible endoscope (3). Therefore, in another preferred embodiment of the present disclosure, said robotic arm (5) further comprises at least one laser fiber actuator (7) for actuating said laser fiber. Said fiber actuator (7) comprises at least one laser fiber holder (16) for holding the laser fiber connection adapter (17) and at least one movement unit (not shown in figures) for moving towards (or moving away) said laser fiber holder (16) to the flexible endoscope (3). Alternatively, basket catheter, forceps, grasper, biopsy catheter, electrode catheter may be used with the robotic manipulator system of the present application.

In another preferred embodiment of the present disclosure, said control unit (2) comprises at least one control console (10). Said control console (10) comprises at least one display device (20) for displaying system parameters (such as velocity of the moving parts, deflection angle of the flexible tip etc.). Control console (10) may further comprises at least one control panel for controlling said system parameters. Users are able to change system parameters using the control panel. In an exemplary embodiment, said control panel may be a key panel. Alternatively, said display device (20) may comprise a touch device so as to perform as a control panel.

European type and USA type for flexible endoscope (3) are different. For example in US type, elevating the deflection lever (18) up deflects the flexible tip of the flexible endoscope (3) up direction, while in the EU type elevating the deflection lever (18) up deflects the flexible tip of the flexible endoscope (3) down direction. Therefore, according to the present disclosure, movement response of the handle lever (14c) is controlled by the control unit (2), preferably by said control panel.

In another preferred embodiment of the present disclosure, said control unit (2) comprises at least one seat (11). Height of said seat (11) is preferably able to be adjusted by the users. Therefore, during the control of the robotic manipulator (1), operator may sit on a comfortable seat (11). Thus, surgical operation becomes much easier for the operator.

In an exemplary embodiment, after insertion of the flexible endoscope (3) into the patient, the flexible endoscope (3) is attached to the robotic manipulator (1). Then, the surgeon sits on to height adjustable and comfortable seat (11) of the control console (2). The height and the distance to knees of the surgeon of the control console (10) can be adjusted for ergonomics from the display device (20) of the console. The position settings according to the surgeon can be stored to memory for future usage. The surgeon can control all functions of the robotic manipulator (1) and all movements of flexible endoscope (3) from the control console (10) by either display device (20) or control means (14). The surgeon can control both fluoroscopy and laser by pressing two foot pedals (13). There is an optional video monitor (12) placed in front of the surgeon to show the images from fluoroscopy or endoscopy camera unit. Normally, it shows the video of endoscopy camera, it switches the fluoroscopy images while the foot-pedal (13) for fluoroscopy is pressed.

In another preferred embodiment, when the endoscope is attached to the manipulator, the deflection lever (18) is placed inside the coupler and actuated precisely. The deflection actuator (6) has a torque control and limiter to prevent any destruction of wire of deflection mechanism.

In another preferred embodiment, robotic arm (5) has a bended shape, so that robotic arm (5) is able to rotate at the central axis (rotation axis) of the flexible endoscope (3). Therefore, during the rotation movement, flexible endoscope (3) do not moves up/down/left/right positions.

In another preferred embodiment, height of the robotic arm (5) is able to be adjusted by at least one vertical movement unit (9). The vertical movement unit enables, height of the robotic arm (5) is adjusted according to position of the patient.

In another exemplary embodiment, the advancement distance of the laser fiber is indicated on the display device (20) as the distance from distal end of the flexible endoscope (3) to the tip of the laser fiber during the movements of laser fiber actuator (7). The software of the robotic manipulator (1) controls the emission of laser by controlling the foot pedal (13). Although the surgeon presses the foot pedal (13) for laser shooting, the robot does not enable the laser if the tip of laser fiber is close to the distal end of flexible endoscope to avoid any damage of endoscope.

In another preferred embodiment, robotic manipulator (1) comprises at least one endoscope holder (24), which comprises said connection unit (4) and which is able to be attached to or detached from said robotic arm (5). Therefore, according to this embodiment, different brand and model of flexible endoscopes (3) are able to be used with present robotic manipulator system. In order to provide attachment/detachment feature, said endoscope holder (24) comprises at least one linear slide (39) and said robotic arm (5) comprises at least one linear slide bearing (38) for receiving said linear slide (39). In order to send control signals coming from control unit (2) through robotic arm (5) to the endoscope holder (24), said robotic arm (5) further comprises signal connector (40) by the linear slide connection.

In another exemplary embodiment, rotation and horizontal (insertion) movement is achieved as follows. The lower plate (36) of horizontal movement is fixed on the top of vertical movement unit (9). Highly precise horizontal movement is achieved by four linear bearings (23) placed between lower plate (36) and upper plate (21). Horizontal motion with variable speed achieved by precisely controlled horizontal movement motor (35) and bearing assembly (30) is transferred by belt and pulley (34). Ball screw nut (33) is connected to ball screw nut flange (31) fixed on the lower plate (36) in order to move the upper plate (21) horizontally. Rotational movement is driven precisely by a rotation motor (29), which is carried by carrier plate (22), connected to a speed reducer (27) (for example a zero backlash Cyclo speed reducer) through a motor flange (28). The speed of rotation is varied according to speed of rotation handle (14b) (according to action of operator). The angle of rotation is measured by a rotation feedback sensor (32). The out shaft of speed reducer (27) is connected to robotic arm (5) through a bearing system (26) and arm flange (25). Said moving mechanism is protected by a cover (37) from external environment.

In another preferred embodiment, the robotic manipulator system comprises at least one irrigation pump (46) as an accessory. That pump is a speed controlled peristaltic pump for pumping the irrigation saline thru working channel of flexible endoscope (3). It can be used for improving vision and operation of laser lithotripsy. The speed of flow rate is adjusted on touch device interface for operator control. The operator can turn on/off the pump from touch device.

In another preferred embodiment, to give realistic feeling to user, the movement on the shaft of the deflection lever (18) is actuated by deflection actuator (6) according to the user control of handle lever (14c) which its motion is transferred to control sensor (42) by a timing belt (44) and the spin of the pulley of that timing belt (44). The force is detected from deflection actuator (6) and that signal is transferred to the controller and timing belt (44) and its pulley are forced by an electro-magnetic brake (43) to give the feeling of any friction and excessive tension on the distal tip of the flexible endoscope. Said timing belt (44) is connected to handle lever (14c) to reflect resistance of the deflection lever (18). Furthermore, the precise control of the deflection is enabled by a precision wheel (41), preferably placed on the middle of the control console (10). When the flexible endoscope (3) is attached to the robotic manipulator (1), the deflection lever (18) is placed inside the coupler and actuated precisely. The deflection actuator (6) has a torque control and limiter to prevent any destruction of tendon wires of deflection mechanism.

## Claims

1. A robotic manipulator system for maneuvering an insertion tube, which is inserted to patient's body, of an existing flexible uretero-renoscope to a desired location within a target zone during a medical procedure, comprising;
- at least one control unit (2) capable of receiving an operator input,
- at least one robotic manipulator (1), in communication with said control unit (2) and responsive to said operator input, said robotic manipulator (1) comprising,
i. at least one controller,
ii. a rotation mechanism in communication with said at least one controller by said input and being rotated about a longitudinal axis of the insertion tube,
iii. a horizontal movement unit (8) in communication with said at least one controller by said input and being displaced along a first path which is parallel or coincide with the longitudinal axis of the insertion tube, and
iv. a deflection actuator (6) capable of receiving a portion of the existing flexible uretero-renoscope, said deflection actuator (6) being in communication with said at least one controller by said input and being displaced along a second path;
wherein displacement of said deflection actuator (6) along said second path causes deflection of a distal end of the existing flexible uretero-renoscope,
- wherein said control unit (2) comprises at least one control console (10) and said control console (10) comprises at least one display device (20) for displaying system parameters,
- wherein the robotic manipulator system comprises at least one laser fiber which is inserted through a working channel of the flexible uretero-renoscope (3), the system further comprising
- a robotic arm (5) of the robotic manipulator (1) further comprises at least one laser fiber actuator (7) for actuating said laser fiber,
- and in that said laser fiber actuator (7) comprises at least one laser fiber holder (16) for holding a laser fiber connection adapter (17) and at least one movement unit for moving said laser fiber holder (16) with respect to the flexible uretero-renoscope (3), and
- wherein an advancement distance of the laser fiber is indicated on the display device (20) as the distance from a distal end of the flexible uretero-renoscope (3) to the tip of the laser fiber during movement of the laser fiber actuator (7), and
- wherein a foot pedal (13) is provided to control emission of laser radiation, and software of the robotic manipulator (1) controls the emission of the laser by controlling the foot pedal (13) such that, when the surgeon presses the foot pedal (13) for laser shooting, the robot does not enable the laser if the tip of the laser fiber is close to the distal end of the flexible uretero-renoscope (3) to avoid any damage of the uretero-renoscope.

2. A robotic manipulator system according to one of the preceding claims, **characterized in that**; said control unit (2) comprises at least one control means (14) for controlling the movement of the robotic manipulator (1).

3. A robotic manipulator system according to one of the preceding claims, **characterized in that**; the robotic arm (5) makes the rotation movement and is in connection with said flexible uretero-renoscope (3), wherein said robotic arm (5) comprises at least one connection unit (4), which fixes said flexible uretero-renoscope (3) to said robotic arm (5).

4. A robotic manipulator system according to one of the preceding claims, **characterized in that**; said horizontal movement unit (8) is placed on the robotic manipulator (1) and moves the robotic manipulator (1) horizontally with respect to the patient, or **in that** said horizontal movement unit (8) is placed on patient's side and moves the patient with respect to the robotic manipulator (1).

5. A robotic manipulator system according to claim 2, **characterized in that**; said control means (14) comprises at least one deflection handle (14a) and at least one rotation and insertion handle (14b).

6. A robotic manipulator system according to claim 5, **characterized in that**; said rotation and insertion handle (14b) is in the form of a stick that both rotates and moves forward/backward.

7. A robotic manipulator system according to claim 5 or 6, **characterized in that**; said deflection handle (14a) comprises at least one handle lever (14c), for performing the deflection movement, wherein preferably movement response of said handle lever (14c) is controlled by said control unit (2) for adapting medical instruments having different standards.

8. A robotic manipulator system according to claim 3, **characterized in that**; said connection unit (4) comprises at least one deflection lever coupler (15) for gripping the deflection lever (18) of the flexible uretero-renoscope (3).

9. A robotic manipulator system according to one of the preceding claims, **characterized in that**; said control console (10) further comprises at least one control panel for controlling system parameters.

10. A robotic manipulator system according to claim 9, **characterized in that**; said control panel is a key panel or said display device (20) comprising a touch panel so as to perform as a control panel.

11. A robotic manipulator system according to one of the preceding claims, **characterized in that**; said robotic manipulator (1) comprises at least one endoscope holder (24), which comprises said connection unit (4) and which is able to be attached to or detached from said robotic arm (5).

12. A robotic manipulator system according to claim 7, **characterized in that**; said handle lever (14c) comprises at least one timing belt (44) for reflecting the resistance of the deflection lever (18).

13. A robotic manipulator system according to claim 12, **characterized in that**; said robotic manipulator (1) comprises at least one control sensor (42) to which the user input given by handle lever (14c) for movement of the timing belt (44) and the robotic manipulator (1) is transmitted.

14. A robotic manipulator system according to one of the preceding claims, **characterized in that**; said robotic manipulator (1) comprises at least one sensing mechanism to detecting friction and excessive tension on the distal tip of the flexible uretero-renoscope (3) from deflection actuator (6).

## Patentansprüche

1. Robotermanipulatorsystem zum Handhaben eines Einführrohrs eines vorhandenen flexiblen Ureter-Renoskops, das in den Körper eines Patienten an einen gewünschten Ort in einem Zielbereich während eines medizinischen Eingriffs eingeführt wird, umfassend:
wenigstens eine zum Empfangen einer Bedienereingabe fähige Steuereinheit (2),
wenigstens einen Robotermanipulator (1) in Verbindung mit der Steuereinheit (2) und reagierend auf die Bedienereingabe, wobei der Robotermanipulator (1) umfasst
i. wenigstens ein Steuergerät,
ii. einen Drehmechanismus in Verbindung mit dem wenigstens einen Steuergerät durch die Eingabe, der um eine Längsachse des Einführrohrs gedreht wird,
iii. eine Horizontalbewegungseinheit (8) in Verbindung mit dem wenigstens einen Steuergerät durch die Eingabe, die entlang einem ersten Weg verschoben wird, der parallel zur Längsachse des Einführrohrs ist oder mit dieser übereinstimmt, und
iv. ein zum Aufnehmen eines Abschnitts des vorhandenen flexiblen Uretero-Renoskops fähigen Biegebetätiger (6), wobei der Biegebetätiger (6) in Verbindung mit dem wenigstens einen Steuergerät durch die Eingabe steht und entlang einem zweiten Weg verschoben wird;
wobei das Verschieben des Biegebetätigers (6) entlang dem zweiten Weg ein Biegen eines distalen Endes des vorhandenen flexiblen Uretero-Renoskops bewirkt,
wobei die Steuereinheit (2) wenigstens eine Steuerkonsole (10) umfasst und wobei die Steuerkonsole (10) wenigstens eine Anzeigevorrichtung (20) zum Anzeigen von Systemparametern umfasst,
wobei das Robotermanipulatorsystem wenigstens eine Laserfaser umfasst, die durch einen Arbeitskanal des flexiblen Uretero-Renoskops (3) eingeführt wird,
wobei das System ferner umfasst
einen Roboterarm (5) des Robotermanipulators (1), der ferner wenigstens einen Laserfaserbetätiger (7) zum Betätigen der Laserfaser umfasst,
und wobei der Laserfaserbetätiger (7) wenigstens einen Laserfaserhalter (16) zum Halten eines Laserfaser-Verbindungsadapters (17) und wenigstens eine Bewegungseinheit zum Bewegen des Laserfaserhalters (16) in Bezug auf das flexible Uretero-Renoskop (3) umfasst, und
wobei ein Vorwärtsbewegungsabstand der Laserfaser an der Anzeigevorrichtung (20) als der Abstand von einem distalen Ende des flexiblen Uretero-Renoskops (3) zum Ende der Laserfaser während der Bewegung des Laserfaserbetätigers (7) angegeben wird und wobei ein Fußpedal (13) zum Steuern des Ausstrahlens von Laserstrahlung angeordnet ist und Software des Robotermanipulators (1) die Ausstrahlung des Lasers durch Steuern des Fußpedals (13) steuert, so dass, wenn der Chirurg das Fußpedal (13) zum Laserbeschuss tritt, der Roboter den Laser nicht aktiviert, wenn sich das Ende der Laserfaser in der Nähe des distalen Endes des flexiblen Uretero-Renoskops (3) befindet, um Schäden am Uretero-Renoskop zu vermeiden.

2. Robotermanipulatorsystem nach einem der vorhergehenden Ansprüche, **dadurch gekennzeichnet, dass** die Steuereinheit (2) wenigstens ein Steuermittel (14) zum Steuern der Bewegung des Robotermanipulators (1) umfasst.

3. Robotermanipulatorsystem nach einem der vorhergehenden Ansprüche, **dadurch gekennzeichnet, dass** der Roboterarm (5) die Drehbewegung ausführt und in Verbindung mit dem flexiblen Uretero-Renoskop (3) steht, wobei der Roboterarm (5) wenigstens eine Verbindungseinheit (4) umfasst, die das flexible Uretero-Renoskop (3) am Roboterarm (5) befestigt.

4. Robotermanipulatorsystem nach einem der vorhergehenden Ansprüche, **dadurch gekennzeichnet, dass** die Horizontalbewegungseinheit (8) am Robotermanipulator (1) angeordnet ist und den Robotermanipulator (1) horizontal in Bezug auf den Patienten bewegt, oder **dadurch gekennzeichnet, dass** die Horizontalbewegungseinheit (8) an der Seite des Patienten angeordnet ist und den Patienten in Bezug auf den Robotermanipulator (1) bewegt.

5. Robotermanipulatorsystem nach Anspruch 2, **dadurch gekennzeichnet, dass** das Steuermittel (14) wenigstens einen Biegegriff (14a) und wenigstens einen Dreh- und Einführgriff (14b) umfasst.

6. Robotermanipulatorsystem nach Anspruch 5, **dadurch gekennzeichnet, dass** der Dreh- und Einführgriff (14b) die Form eines Stabs aufweist, der sich dreht und vorwärts/rückwärts bewegt.

7. Robotermanipulatorsystem nach Anspruch 5 oder 6, **dadurch gekennzeichnet, dass** der Biegegriff (14a) wenigstens einen Griffhebel (14c) zum Durchführen der Biegebewegung umfasst, wobei die Bewegungsreaktion des Griffhebels (14c) vorzugsweise durch die Steuereinheit (2) zur Anpassung am medizinische Instrumente mit verschiedenen Standards gesteuert wird.

8. Robotermanipulatorsystem nach Anspruch 3, **dadurch gekennzeichnet, dass** die Verbindungseinheit (4) wenigstens einen Biegehebelkoppler (15) zum Greifen des Biegehebels (18) des flexiblen Uretero-Renoskop (3) umfasst.

9. Robotermanipulatorsystem nach einem der vorhergehenden Ansprüche, **dadurch gekennzeichnet, dass** die Steuerkonsole (10) ferner wenigstens ein Bedienfeld zum Steuern von Systemparametern umfasst.

10. Robotermanipulatorsystem nach Anspruch 9, **dadurch gekennzeichnet, dass** das Bedienfeld ein Tastenfeld ist oder die Anzeigevorrichtung (20) einen Touchscreen umfasst, der als Bedienfeld dient.

11. Robotermanipulatorsystem nach einem der vorhergehenden Ansprüche, **dadurch gekennzeichnet, dass** der Robotermanipulator (1) wenigstens einen Endoskophalter (24) umfasst, der die Verbindungseinheit (4) umfasst und der am Roboterarm (5) befestigt oder von diesem abgenommen werden kann.

12. Robotermanipulatorsystem nach Anspruch 7, **dadurch gekennzeichnet, dass** der Griffhebel (14c) wenigstens einen Steuerriemen (44) zum Widerspiegeln des Widerstands des Biegehebels (18) umfasst.

13. Robotermanipulatorsystem nach Anspruch 12, **dadurch gekennzeichnet, dass** der Robotermanipulator (1) wenigstens einen Steuersensor (42) umfasst, an den die vom Griffhebel (14c) für die Bewegung des Steuerriemens (44) und des Robotermanipulators (1) gelieferte Benutzereingabe gesendet wird.

14. Robotermanipulatorsystem nach einem der vorhergehenden Ansprüche, **dadurch gekennzeichnet, dass** der Robotermanipulator (1) wenigstens einen Erfassungsmechanismus zum Erfassen von Reibung und zu starker Spannung am distalen Ende des flexiblen Uretero-Renoskops (3) vom Biegebetätiger (6) umfasst.

## Revendications

1. Système manipulateur robot pour manœuvrer un tube d'insertion, qui est inséré au niveau d'un corps d'un·e patient·e d'un urétérorénoscope souple existant au niveau d'un emplacement souhaité à l'intérieur d'une zone cible durant une procédure médicale, comprenant :
- au moins une unité de commande (2) capable de recevoir une entrée d'opérateur·trice,
- au moins un manipulateur robot (1), en communication avec ladite unité de commande (2) et sensible à ladite entrée d'opérateur·trice, ledit manipulateur robot (1) comprenant,
i. au moins un dispositif de commande,
ii. un mécanisme de rotation en communication avec ledit au moins un dispositif de commande par ladite entrée et étant tourné autour d'un axe longitudinal du tube d'insertion,
iii. une unité de mouvement horizontal (8) en communication avec ledit au moins un dispositif de commande par ladite entrée et étant déplacée le long d'un premier trajet qui est parallèle ou qui coïncide avec l'axe longitudinal du tube d'insertion, et
iv. un dispositif d'actionnement de déflection (6) capable de recevoir une partie de l'urétérorénoscope souple existant, ledit dispositif d'actionnement de déflection (6) se trouvant en communication avec ledit au moins un dispositif de commande par ladite entrée et étant déplacé le long d'un second trajet ;
où le déplacement dudit dispositif d'actionnement de déflection (6) le long dudit second trajet amène la déflection d'une extrémité distale de l'urétérorénoscope souple existant,
- ladite unité de commande (2) comprenant au moins une console de commande (10) et ladite console de commande (10) comprenant au moins un dispositif d'affichage (20) pour l'affichage des paramètres du système,
- le système manipulateur robot comprenant au moins une fibre laser qui est insérée à travers un canal de travail de l'urétérorénoscope souple (3), le système comprenant en outre
- un bras robotique (5) du manipulateur robot (1) comprenant en outre au moins un dispositif d'actionnement de fibre laser (7) pour l'actionnement de ladite fibre laser,
- et en ce que ledit dispositif d'actionnement de fibre laser (7) comprend au moins un dispositif de retenue de fibre laser (16) pour retenir un adaptateur de connexion de fibre laser (17) et au moins une unité de mouvement pour déplacer ledit dispositif de retenue de fibre laser (16) par rapport à l'urétérorénoscope souple (3), et
- une distance d'avancement de la fibre laser est indiquée sur le dispositif d'affichage (20) comme distance depuis une extrémité distale de l'urétérorénoscope souple (3) vers la pointe de la fibre laser durant le mouvement du dispositif d'actionnement de fibre laser (7), et
- une pédale à pied (13) est disposée pour commander l'émission du rayonnement laser, et le logiciel du manipulateur robot (1) commande l'émission du laser en commandant la pédale à pied (13) de sorte que, lorsque le·la chirurgien·ienne appuie sur la pédale à pied (13) pour le tir laser, le robot n'active pas le laser si la pointe de la fibre laser est proche de l'extrémité distale de l'urétérorénoscope souple (3) pour éviter toute détérioration de l'urétérorénoscope.

2. Système manipulateur robot selon l'une des revendications précédentes, **caractérisé en ce que**, ladite unité de commande (2) comprend au moins un moyen de commande (14) pour commander le mouvement du manipulateur robot (1).

3. Système manipulateur robot selon l'une des revendications précédentes, **caractérisé en ce que**, le bras robotique (5) effectue le mouvement de rotation et se trouve en connexion avec ledit urétérorénoscope souple (3), ledit bras robotique (5) comprenant au moins une unité de connexion (4), qui fixe ledit urétérorénoscope souple (3) audit bras robotique (5).

4. Système manipulateur robot selon l'une des revendications précédentes, **caractérisé en ce que**, ladite unité de mouvement horizontal (8) est placée sur le manipulateur robot (1) et déplace le manipulateur robot (1) horizontalement par rapport au ·à la patient·e, ou **en ce que** ladite unité de mouvement horizontal (8) est placée sur le côté du·de la patient·e et déplace le·la patient·e par rapport au manipulateur robot (1).

5. Système manipulateur robot selon la revendication 2, **caractérisé en ce que**, ledit moyen de commande (14) comprend au moins une poignée de déflection (14a) et au moins une poignée de rotation et d'insertion (14b).

6. Système manipulateur robot selon la revendication 5, **caractérisé en ce que**, ladite poignée de rotation et d'insertion (14b) se présente sous la forme d'une tige qui à la fois tourne et se déplace vers l'avant/vers l'arrière.

7. Système manipulateur robot selon la revendication 5 ou 6, **caractérisé en ce que**, ladite poignée de déflection (14a) comprend au moins un levier de poignée (14c), pour effectuer le mouvement de déflection, préférablement la réponse de mouvement dudit levier de poignée (14c) étant commandée par ladite unité de commande (2) pour l'adaptation d'instruments médicaux ayant des normes différentes.

8. Système manipulateur robot selon la revendication 3, **caractérisé en ce que**, ladite unité de connexion (4) comprend au moins un dispositif d'accouplement de levier de déflection (15) pour saisir le levier de déflection (18) de l'urétérorénoscope souple (3).

9. Système manipulateur robot selon l'une des revendications précédentes, **caractérisé en ce que**, ladite console de commande (10) comprend en outre au moins un panneau de commande pour commander les paramètres du système.

10. Système manipulateur robot selon la revendication 9, **caractérisé en ce que**, ledit panneau de commande est un panneau de touches ou ledit dispositif d'affichage (20) comprend un panneau tactile afin de se comporter comme panneau de commande.

11. Système manipulateur robot selon l'une des revendications précédentes, **caractérisé en ce que**, ledit manipulateur robot (1) comprend au moins un dispositif de retenue d'endoscope (24), qui comprend ladite unité de connexion (4) et qui est capable d'être fixé ou détaché dudit bras robotique (5).

12. Système manipulateur robot selon la revendication 7, **caractérisé en ce que**, ledit levier de poignée (14c) comprend au moins une courroie de synchronisation (44) pour refléter la résistance du levier de déflection (18).

13. Système manipulateur robot selon la revendication 12, **caractérisé en ce que**, ledit manipulateur robot (1) comprend au moins un détecteur de commande (42) auquel l'entrée utilisateur·trice donnée par le levier de poignée (14c) pour le mouvement de la courroie de synchronisation (44) et du manipulateur robot (1) est transmise.

14. Système manipulateur robot selon l'une des revendications précédentes, **caractérisé en ce que**, ledit manipulateur robot (1) comprend au moins un mécanisme de détection pour détecter le frottement et la tension excessive sur la pointe distale de l'urétérorénoscope souple (3) provenant du dispositif d'actionnement de déflection (6).
